# EUROPEAN PATENT SPECIFICATION

(11) **EP 1 539 056 B1**
(45) Date of publication and mention of the grant of the patent: **18.05.2011**
(21) Application number: 03719775.3
(22) Date of filing: 15.04.2003
(51) Int. Cl.: A61F 5/00

(54) **PREFORMED ANKLE BRACE**
VORGEFORMTE FUSSGELENKSCHIENE
ATTELLE POUR CHEVILLE PREFORMEE

(43) Date of publication of application: 15.06.2005
(73) Proprietor: BSN Medical, Inc., Wilmington, DE 19801 (US)
(72) Inventor: EVANS, John, C., Lancashire OL 16 3RF (GB); O'HARA, Martin, Charlotte, NC 28277 (US)
(74) Representative: Sherrard-Smith, Hugh
(86) International application number: PCT/US2003/011709
(87) International publication number: WO 2004/098467

(56) References cited:
- WO-A-98/08470
- US-A- 5 125 400
- US-A- 5 389 065
- US-B1- 6 406 450

## Description

WO 98/08470 discloses a thermoplastic pad that has a thermoplastic overmold of the same material as the pad overlapping in a fitting manner the edge of the pad.

### Technical Field and Background of the Invention

This invention relates to a preformed ankle brace that includes soft edge portions. The ankle brace also includes novel inner layers that provide comfort, cleanliness and washability in a lightweight, preformed brace product. The brace provides support to the lateral and medial aspects of the ankle and lower leg and stabilizes the ankle against inversion and eversion, while permitting normal dorsiflexion and plantarflexion of the ankle during healing. The brace is specifically designed to be worn with a shoe and is thus dimensionally configured to fit within a shoe.

The brace provides enhanced comfort by conforming to the medial and lateral aspects of the ankle and lower leg while providing edge portions that prevent contact between the skin and the hard shell portions of the brace.

The brace is simple, robust, and can be manufactured with relatively few manufacturing steps.

### Summary of the Invention

Therefore, it is an object of the invention to provide an ankle brace that includes soft edge portions.

It is another object of the invention to provide an ankle brace that includes inner padding that is comfortable and easy to clean.

It is another object of the invention to provide an ankle brace that includes both hard shell support elements and padding elements.

It is another object of the invention to provide an ankle brace that has an inner padding layer of variable thickness.

These and other objects of the present invention are achieved in the preferred embodiments disclosed below by providing an ankle brace adapted to extend along the lateral and medial aspects of an injured ankle and lower leg for supporting the ankle during healing. A medial side support is provided for extending along the medial aspect of the ankle and lower leg. The medial side support includes an elongate, rigid support member having a lower end of sufficiently narrow width and thinness to fit inside a shoe being worn by a wearer of the brace. The medial side support includes a soft, flexible perimeter edge portion mechanically adhered to and extending outwardly in overlapping, non-interfitting relation to the medial support member for protecting the medial aspect of the leg and ankle from irritation resulting from contact with edges of the support member. A lateral side support extends along the lateral aspect of the ankle and lower leg, the lateral side support includes an elongate, rigid support member having a lower end of sufficiently narrow width and thinness to fit inside the shoe. The lateral support member includes a soft, flexible perimeter edge portion mechanically adhered to and extends outwardly in overlapping, non-interfitting relation to the support member for protecting the lateral aspect of the leg and ankle from irritation resulting from contact with edges of the rigid support member. Connector means connect the medial side support and lateral side support together at their respective lower ends and extend under the foot of the wearer of the brace.

According to one preferred embodiment of the invention, the medial side support and the lateral side support each include a padding material positioned adjacent an inner face of the respective rigid support members.

According to another preferred embodiment of the invention, the perimeter edge portions are adhered to the respective rigid support members by an adhesive.

According to yet another preferred embodiment of the invention, the padding material overlaps and extends outwardly beyond side edges of the respective side support members, and the perimeter edge portions overlap and extend outwardly beyond side edges of both the support members and the padding material.

According to yet another preferred embodiment of the invention, the medial rigid support members and the lateral rigid support member each are formed of a preformed plastic material.

According to yet another preferred embodiment of the invention, the padding material overlaps and extends outwardly beyond the side edges of the rigid support members. The perimeter edge portions overlap and extend outwardly beyond the side edges of both the rigid support members and the padding material, and the perimeter edge portions are adhered to both the rigid support members and to overlapped portions of the padding material.

Preferably, the padding material comprises a three-dimensional fabric.

According to yet another preferred embodiment of the invention, the padding material comprises two overlaid layers of a three-dimensional fabric.

According to yet another preferred embodiment of the invention, the padding material comprises at least one layer of a three-dimensional fabric of the type characterized by being highly air-permeable and free of latex.

According to yet another preferred embodiment of the invention, the brace includes at least one strap for retaining the medial side support and lateral side support in a closely-conforming condition against the lower leg and ankle.

According to yet another preferred embodiment of the invention, the padding material is between 6 and 12 mm thick.

According to yet another preferred embodiment of the invention, the padding material is 9 mm thick.

According to yet another preferred embodiment of the invention, the padding material comprises a single layer overlying the inner face of the rigid support member, and a second layer overlying an area of the rigid support member adapted to reside next to and support the ankle.

According to yet another preferred embodiment of the invention, the connector means comprises a medial strap carried by the medial support member, a lateral strap carried by the lateral support member, and complementary attachment members carried by the medial strap and lateral strap, respectively, for permitting the medial strap and the lateral strap to be releasably connected.

According to yet another preferred embodiment of the invention, the complementary attachment members comprise hook and loop fastener assemblies.

According to yet another preferred embodiment of the invention, the connector means comprises a strap carried by one of the medial support member or lateral support member having an attachment member carried on one end thereof, and a complementary attachment member carried on an outer surface of the other of the medial support member and the lateral support member for cooperating with the attachment member for connecting the medial side support and the lateral side support.

According to yet another preferred embodiment of the invention, the perimeter edge portion is selected from the group consisting of vinyl, rubber or synthetic rubber.

According to yet another preferred embodiment of the invention, the perimeter edge portion is tapered towards the outer edge thereof to increase flexibility.

### Brief Description of the Drawings

Some of the objects of the invention have been set forth above. Other objects and advantages of the invention will appear as the invention proceeds when taken in conjunction with the following drawings, in which:

Figure 1 is a perspective view of an ankle brace according to one embodiment of the invention;

Figure 2 is a perspective view with parts broken away of a side support of the brace;

Figure 3 is a cross-sectional view taken through line 3--3 of Figure 2;

Figures 4-7 are perspective, medial, front and lateral views of the brace according to Figures 1-3 in place on a foot;

Figure 8 is a perspective view of an ankle brace according to another embodiment of the invention;

Figure 9 is a perspective view of an ankle brace according to yet another embodiment of the invention;

Figure 10 is a perspective view of the ankle brace according to Figure 9 in position to be placed on the ankle leg of the wearer;

Figure 11 is a perspective view of an ankle brace according to yet another embodiment of the invention;

Figure 12 is a perspective view of an ankle brace according to yet another embodiment of the invention; and

Figure 13 is a cross-sectional view taken through line 13--13 of Figure 12.

### Description of the Preferred Embodiment and Best Mode

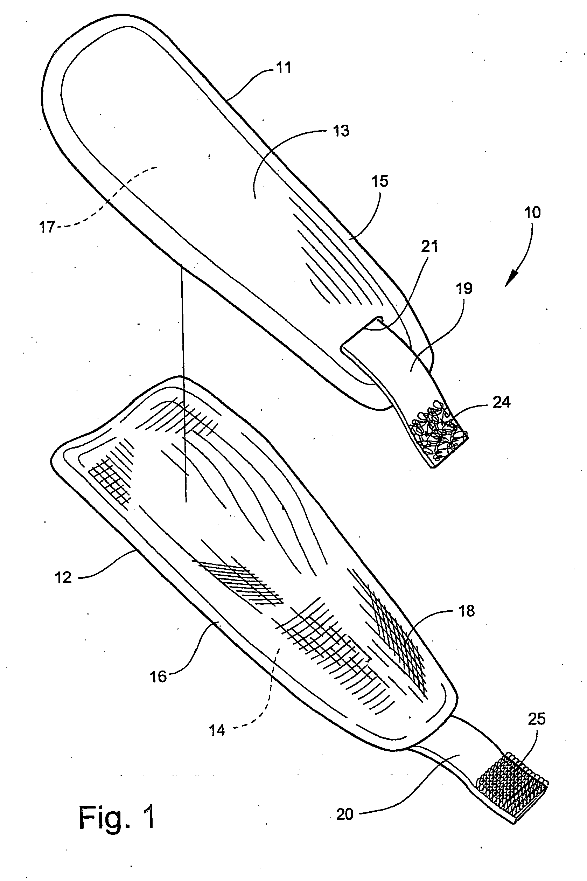

Referring now specifically to the drawings, a ankle brace according to the present invention is illustrated in Figure 1 and shown generally at reference numeral 10. Ankle brace 10 is formed of a pair of side supports 11 and 12. The side supports 11 and 12 are preferably identical so that either can be used on the medial and lateral aspects of the lower leg and ankle.

The side supports 11 and 12 are formed of elongate rigid support members 13 and 14, respectively, each having a lower end of sufficiently narrow width and thinness to fit inside a shoe being worn by a wearer of the brace. The support members 13, 14 are preferably molded of thermoplastic material and are formed in a generally curved shape along the longitudinal axis so as to present an elongate concave surface to the lower leg and ankle being supported. Suitable materials include 12 melt polypropylene that is approximately 1.3 mm thick. The outside surface is preferably polished.

The side supports 11 and 12 also include respective soft, flexible perimeter edge portions 15, 16 mechanically adhered to and extending outwardly in overlapping, non-interfitting relation to the support members 13, 14 for protecting the medial aspect of the leg and ankle from irritation resulting from contact with edges of the rigid support member 13, 14. Suitable materials include vinyl, rubber, or thermoplastic elastomer such as that sold by Advanced Elastomer Systems under the trademark Santoprene®.

The inner, concave surface of the side supports 11 and 12 is covered with a padding material 17, 18, respectively, for protecting the lower leg and ankle from contact with the major concave surface of the rigid support members 13, 14. As is shown in Figure 1, the padding material 17, not shown, and also 18, covers the full perimetrical extent of the side support members 13,14 and thus provides full protection against contact except at the edges. The padding 17, 18 preferably does not extend outwardly to the outer perimeter of the flexible edge portions 15, 16, also shown in Figure 1. Rather, the edge portions 15, 16 are left exposed and will typically be spaced from contact with the lower leg and ankle by at least some of the thickness of the padding material 17, 18. As movement occurs and the padding material 17, 18 is compressed, contact with the rigid support members 13, 14 is prevented by the flexible edge portions 15, 16, respectively.

A preferred padding material is a three-dimensional spacer fabric, such as a knitted spacer fabric manufactured by Tytex Group. Such fabrics are extremely light weight but nevertheless provide a robust cushioning effect for any given thickness and density. They serve well as substitutes for foam, elastic and neoprene, and are latex-free. Moisture transfer and high air permeability provide additional benefits in this particular application. Typical thicknesses of the padding material 17, 18 are in the range of 6-12 mm, with one preferred thickness being two layers of 4.5 mm, for a total of 9 mm. These thicknesses may be obtained by use of a single layer or multiple layers that collectively produce the desired thickness. The padding material 17, 18 is easily cut to shape by die-cutting, sonic cutting and welding or other suitable means, and is easily adhered to the concave inner face of the support members 11, 12 with any of a wide variety of known adhesives approved for use in medical applications. Alternatively, the padding material may be releasably adhered to the concave innerface of the side supports 11, 12 with attachment members such as hook-and-loop or other types of touch fasteners, not shown. Releasability provides the option of increasing or decreasing the thickness, density, air permeability or other characteristics of the padding, or replacing worn padding material 17, 18, while retaining the side supports 11 and 12.

The side supports 11 and 12 are connected and held in place in the shoe by a connector means, such as a pair of woven, knitted, nonwoven fabric or plastic straps 19, 20. As shown in Figure 1, one manner of attaching the straps 19, 20 is to insert and attach one end of the strap 19 and 20 within a slot, shown by way of example at 21 on side support 11. The free end of the straps 19, 20 are provided with complementary patches of loops 24 and hooks 25. The patches of loops and hooks 24, 25 are sufficiently long that adjustment can be accomplished by the degree of overlap between the respective patches of loops and hooks 24, 25 while still providing a connection with adequate resistance to separation. As noted below, numerous other connection methods are possible.

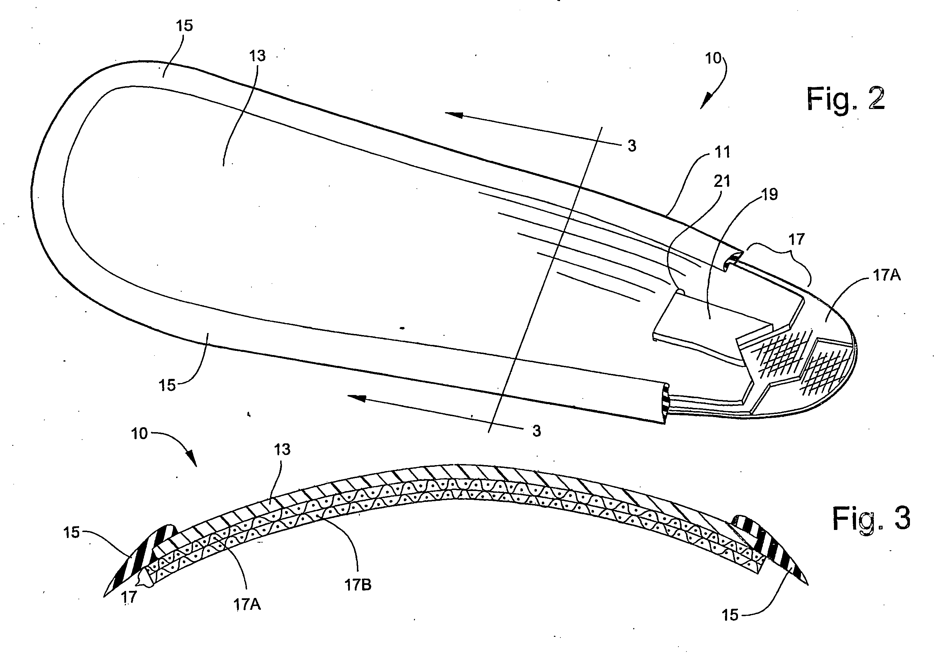

Referring now to Figures 2 and 3, reference will be made to the side support 11 as exemplary of both side supports 11 and 12. The padding material 17 of side support 11 includes two layers 17A, 17B, of fabric padding material 17A, 17B underlying the rigid side support member 13 and an inner portion of the flexible edge portion 15. As best shown in Figure 3, the side support 11 comprises an adhered lamination of the side support member 13 and the two layers of fabric padding material 17A, 17B. The flexible edge portion 15 is preferably mechanically adhered to both the side support member 13 and the underlying padding material layers 17A, 17B in an overlapping, non-interfering relation to the side support member 13. The flexible edge portion 15 is preferably adhered by a suitable adhesive.

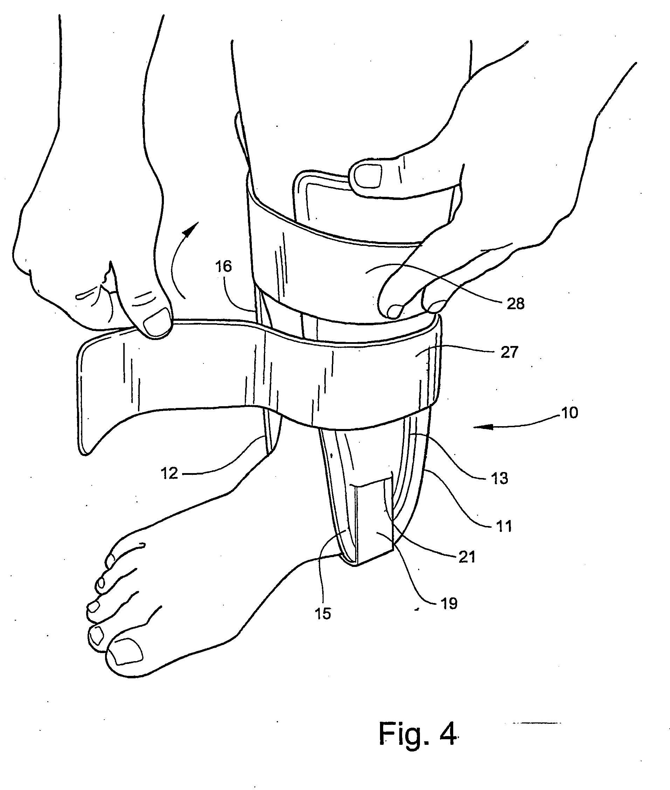

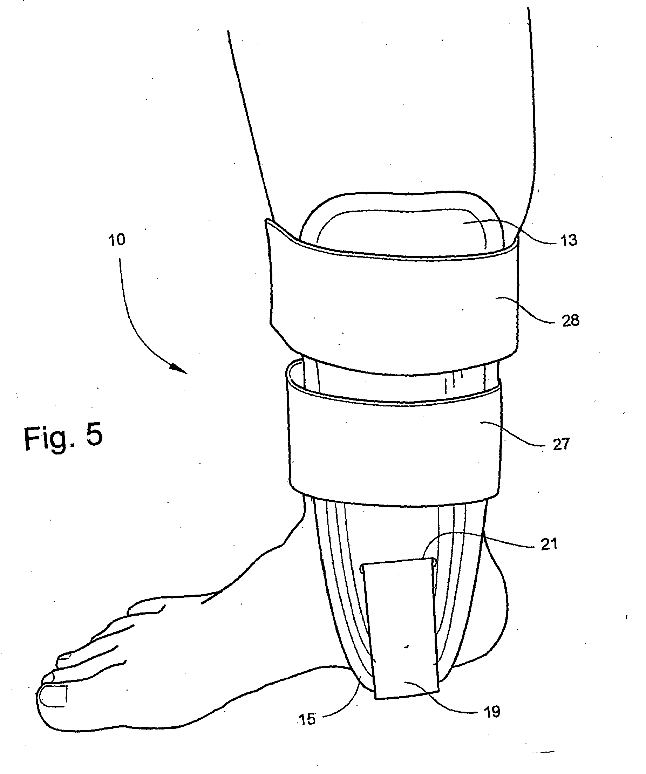

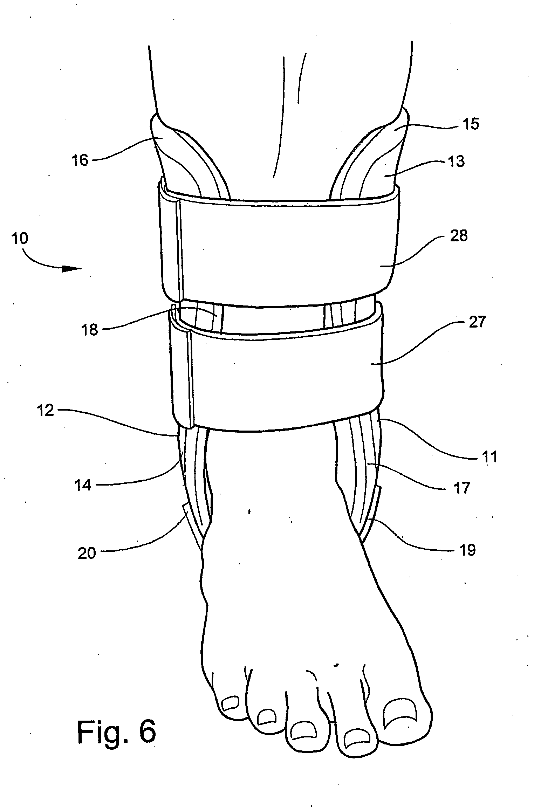

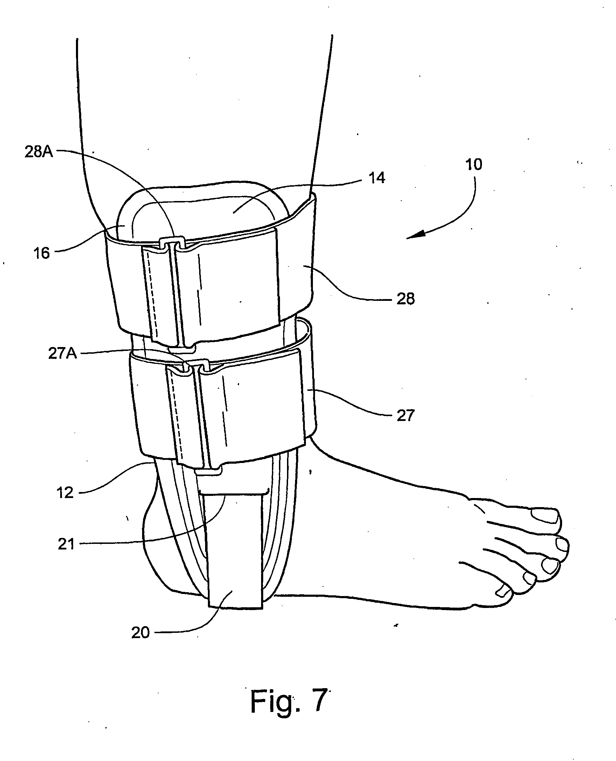

Referring now Figures 4-7, application of the brace 10 is straightforward, and includes attaching the straps 19 and 20, placing the attached straps 19, 20 under the heel, folding the side supports 11 and 12 up against the medial and lateral aspects, respectively, of the lower leg and ankle. The straps 19 and 20 are adjusted as needed. The brace 10 is then secured to the lower leg and ankle with, for example, straps 27, 28. Straps 27, 28 may be either elastic or inelastic, and typically may include complementary hook and loop material to secure the straps 27, 28 in the desired position. As best shown in Figure 7, the straps 27, 28 may include buckles 27A, 28A, respectively to assist in adjusting and securing the straps in place. Alternatively, an elastic wrap, not shown, may be used. The brace 10 may be worn either over or under a sock.

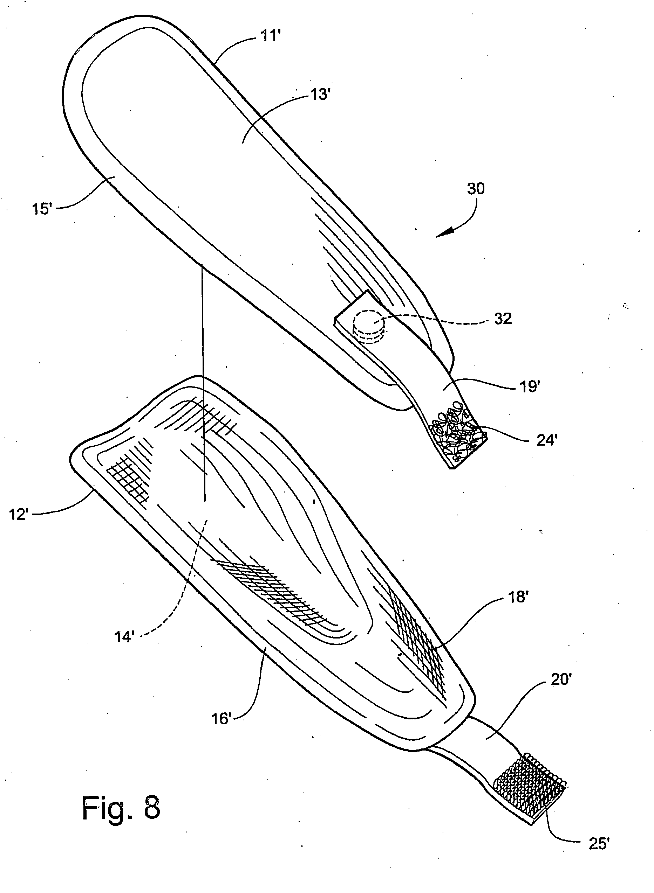

Referring now to Figure 8, a brace 30 is shown, prime notation indicating elements in common with Figures 1-7. The straps 19' and 20' are secured to the rigid support members 13 and 14 by a pivotable rivet 32, one shown on the support member 13. The rivet 32 requires only a small hole instead of a slot, and allows the straps 19', 20' to rotate if necessary to accommodate the size and shape of the foot in relation to the lower leg.

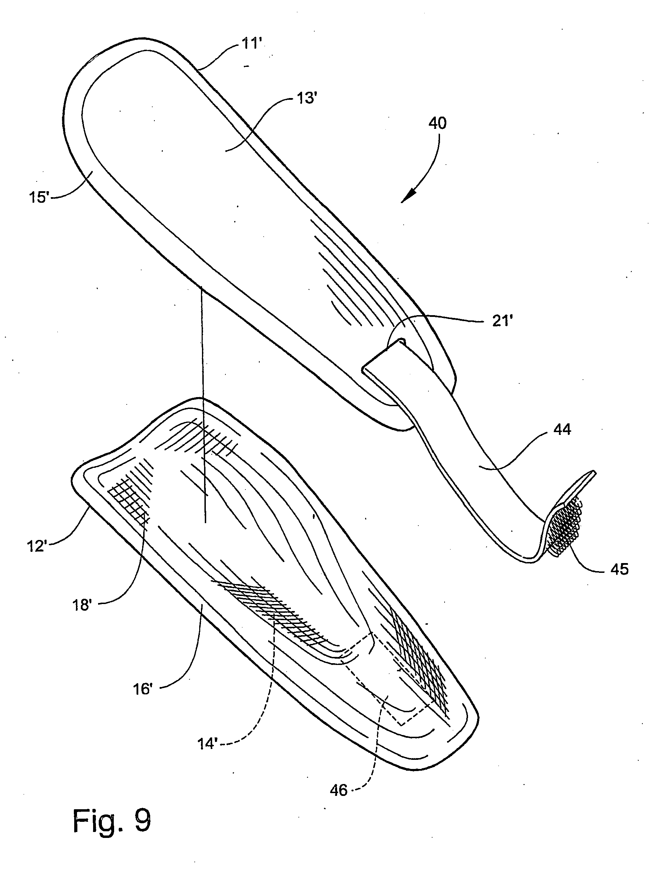

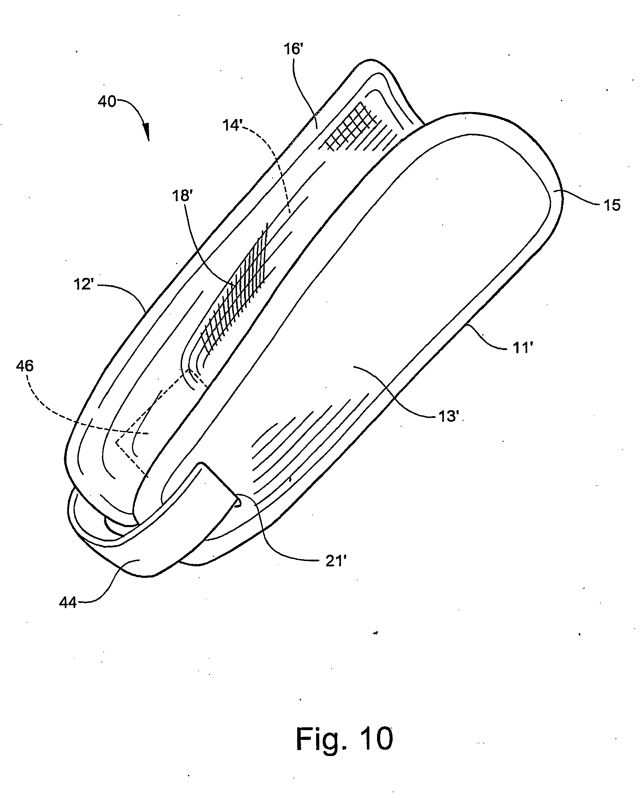

Referring now to Figures 9 and 10, a brace 40 is shown, prime notation indicating elements in common with Figures 1-7. A strap 44 is secured to the rigid support members 13' and 14' by insertion into a slot 21', and attachment to the rigid support member 13' within the slot 21'. The strap 44 is sufficiently long to extend under the foot and up opposing side of the ankle to attach with a mating patch of complementary attachment material. For example, by placement of a patch of hook material 45 on one face of the strap 44, the strap 44 mates with a complementary patch of loop material 46 on the outer face of the support member 14'. The length of the patch of hook material 45 and the loop material 45 is sufficiently long to permit adjustment to the extent necessary to accommodate a predetermined size range. Of course, the rivet 32 shown in Figure 8 could be used on the embodiment shown in Figures 8 and 9, as well.

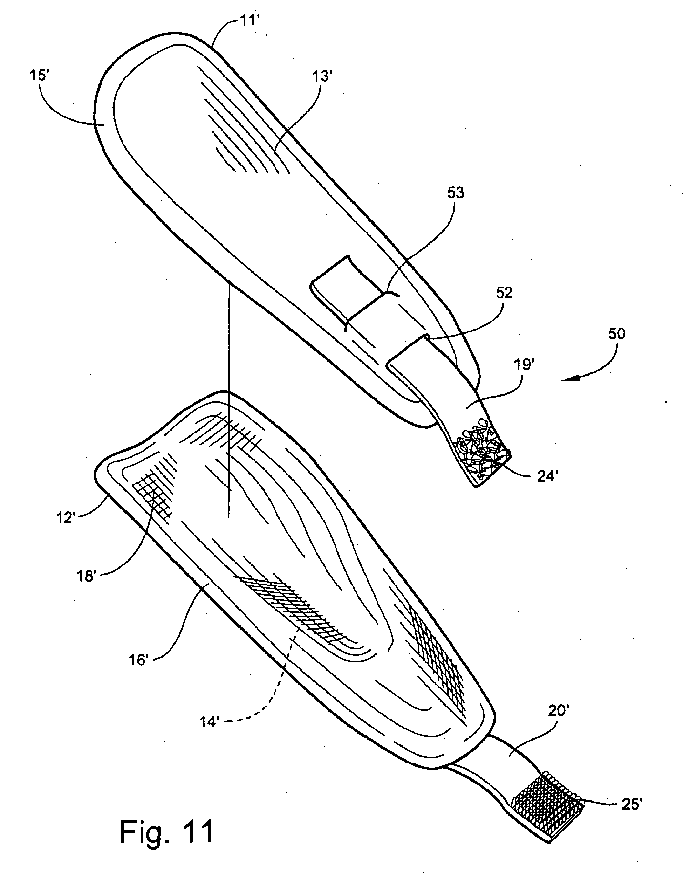

Referring now to Figure 11, a brace 50 is shown, prime notation indicating elements in common with Figures 1-7. The straps 19' and 20' are secured to the rigid support members 13' and 14' by a means of a pair of spaced-apart slots 52, 53, shown on the support member 13'. The straps 19', 20' and the slots 52, 53 are sized so that the straps 19', 20' are frictionally-retained at a given position, thus permitting adjustment by the wearer by pulling or pushing the straps 19', 20' as required for proper adjustment and comfort without removing the brace 50.

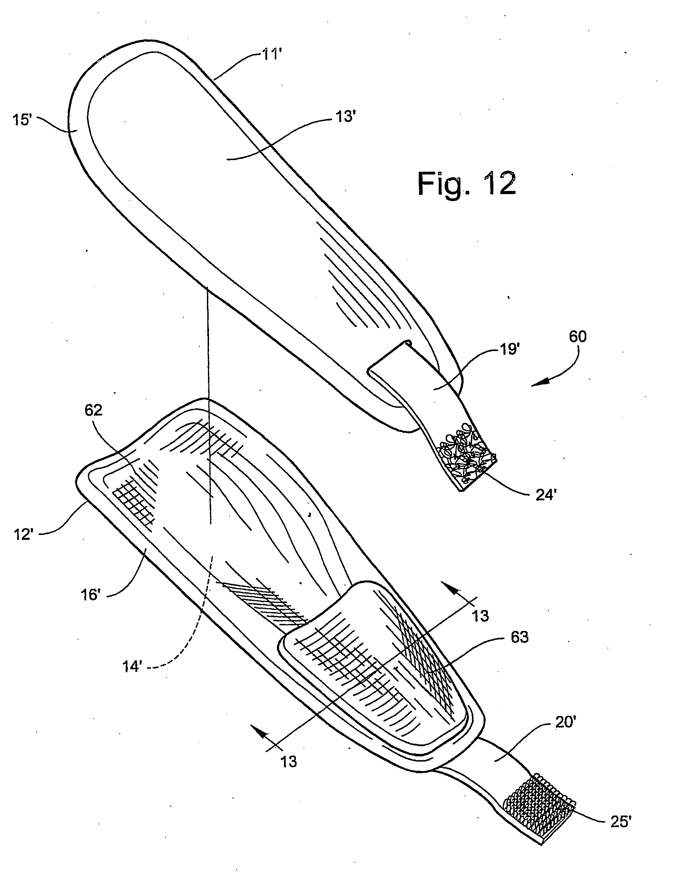

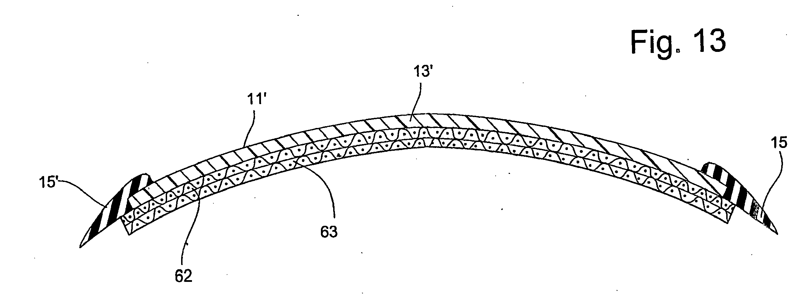

Referring now to Figures 12 and 13, a brace 60 according to a further embodiment of the invention is shown, prime notation indicating elements in common with Figures 1-7. The padding material is provided in a single layer 62 that covers the full perimetrical extent of the side support members 13', 14' and thus provides full protection against contact except at the edges. A second layer of padding material 63 is shaped and sized to fit onto the area of the ankle defined by the medial malleolus and lateral malleolus, thus providing additional protection for these prominences.

A ankle brace is described above.' Various details of the invention may be changed without departing from its scope. Furthermore, the foregoing description of the preferred embodiment of the invention and the best mode for practicing the invention are provided for the purpose of illustration only and not for the purpose of limitation--the invention being defined by the claims.

## Claims

1. An ankle brace adapted to extend along the lateral and medial aspects of an injured ankle and lower leg for supporting the ankle during healing, and comprising:
a medial side support (11) for extending along the medial aspect of the lower leg,
a lateral side support (12) for extending along the lateral aspect of the ankle and lower leg, and
connector means (19, 20) for connecting the medial side support and lateral side support together at their respective lower ends and extending under the foot of the wearer of the brace;
**characterized in that**
(a) the medial side includes an elongate, rigid support member (13) having a lower end of sufficiently narrow width and thinness to fit inside a shoe being worn by a wearer of the brace, said medial side support including a soft, flexible perimeter edge portion (15) mechanically adhered to and extending outwardly in overlapping, non-interfitting relation to the rigid support member for protecting the medial aspect of the leg and ankle from irritation resulting from contact with edges of the rigid support member, and
(b) the lateral side support includes an elongate, rigid support member (14) having a lower end of sufficiently narrow width and thinness to fit inside the shoe, said lateral side support including a soft, flexible perimeter edge portion (16) mechanically adhered to and extending outwardly in overlapping, non-interfitting relation to the rigid support member for protecting the lateral aspect of the leg and ankle from irritation resulting from contact with edges of the rigid support member.

2. An ankle brace according to claim 1, wherein the medial side support and the lateral side support each include a padding material (17,18) positioned adjacent an inner face of the respective rigid support members.

3. An ankle brace according to claim 1, wherein the perimeter edge portions are adhered to the respective rigid support members by an adhesive.

4. An ankle brace according to claim 3, wherein the perimeter edge portions are adhered to an outer facing surface of the respective medial and lateral side supports.

5. An ankle brace according to claim 1, wherein the padding material overlaps and extends outwardly beyond side edges of the respective rigid support members, and the perimeter edge portions overlap and extend outwardly beyond side edges of both the rigid support members and the padding material.

6. An ankle brace according to claim 1, 2, 3, 4 or 5, wherein the medial support member and the lateral support member each are formed of a preformed plastic material.

7. An ankle brace according to claim 5, wherein:
(a) said padding material overlaps and extends outwardly beyond the side edges of the rigid support members;
(b) said perimeter edge portions overlap and extend outwardly beyond the side edges of both the rigid support member and the padding material; and
(c) said perimeter edge portions are adhered to both the rigid side support and to overlapped portions of the padding material.

8. An ankle brace according to claim 6, wherein said padding material comprises a three-dimensional fabric.

9. An ankle brace according to claim 6, wherein said padding material comprises two overlaid layers of a three-dimensional fabric.

10. An ankle brace according to claim 6, wherein said padding material comprises at least one layer of a three-dimensional fabric of the type **characterized by** being highly air-permeable and free of latex.

11. An ankle brace according to claim 1, and including at least one strap for retaining the medial side support and lateral side support in a closely-conforming condition against the lower leg and ankle.

12. An ankle brace according to claim 10, wherein said padding material is between 6 and 12 mm thick.

13. An ankle brace according to claim 10, wherein said padding material is 9 mm thick.

14. An ankle brace according to claim 2, wherein the padding material comprises:
(a) a single layer overlying the inner face of the rigid support members; and
(b) a second layer overlying an area of the rigid support member adapted to reside next to and support the ankle.

15. An ankle brace according to claim 14, wherein the padding material comprises a three-dimensional fabric.

16. An ankle brace according to claim 1, wherein said connector means comprises:
(a) a medial strap carried by the medial support member;
(b) a lateral strap carried by the lateral support member; and
(c) complementary attachment members carried by the medial strap and lateral strap, respectively, for permitting the medial strap and the lateral strap to be releasably connected.

17. An ankle brace according to claim 16, wherein said complementary attachment members comprise hook and loop fastener assemblies.

18. An ankle brace according to claim 1, wherein said connector means comprises:
(a) a strap carried by one of the medial support member or lateral support member having an attachment member carried on one end thereof;
(b) a complementary attachment member carried on an outer surface of the other of the medial support member and the lateral support member for cooperating with the attachment member for connecting the medial side support and the lateral side support.

19. An ankle brace according to claim 1, wherein said perimeter edge portion is selected from the group consisting of vinyl, rubber or synthetic rubber.

20. An ankle brace according to claim 1, wherein said perimeter edge portion is tapered towards the outer edge thereof to increase flexibility.

## Patentansprüche

1. Fußgelenkschiene, die dafür eingerichtet ist, sich entlang dem Lateralaspekt und dem Medialaspekt eines verletzten Fußgelenks und des unteren Beins zu erstrecken, um das Fußgelenk während des Heilens zu stützen, welche aufweist:
eine Medialseitenstütze (11), die sich entlang dem Medialaspekt des unteren Beins erstreckt,
eine Lateralseitenstütze (12), die sich entlang dem Lateralaspekt des Fußgelenks und des unteren Beins erstreckt, und
Verbindungsmittel (1 9 , 20) zum Verbinden der Medialseitenstütze und der Lateralseitenstütze miteinander an ihren jeweiligen unteren Enden, welche sich unter dem Fuß des Trägers der Schiene erstrecken,
**dadurch gekennzeichnet, dass**
(a) die Medialseitenstütze ein lang gestrecktes, steifes Stützelement (13) aufweist, das ein unteres Ende mit einer ausreichend geringen Breite und Dünnheit aufweist, um in einen von einem Träger der Schiene getragenen Schuh zu passen, wobei die Medialseitenstütze einen weichen, flexiblen Umfangsrandabschnitt (15) aufweist, der mechanisch an dem steifen Stützelement haftet und sich in einer überlappenden, nicht einpassenden Beziehung zu dem steifen Stützelement nach außen erstreckt, um den Medialaspekt des Beins und des Fußgelenks vor Reizungen infolge eines Kontakts mit den Rändern des steifen Stützelements zu schützen, und
(b) die Lateralseitenstütze ein längliches, steifes Stützelement (14) aufweist, das ein unteres Ende mit einer ausreichend geringen Breite und Dünnheit aufweist, um in den Schuh zu passen, wobei die Lateralseitenstütze einen weichen, flexiblen Umfangsrandabschnitt (16) aufweist, der mechanisch an dem steifen Stützelement haftet und sich in einer überlappenden, nicht einpassenden Beziehung zu dem steifen Stützelement nach außen erstreckt, um den Lateralaspekt des Beins und des Fußgelenks vor Reizungen infolge eines Kontakts mit den Rändern des steifen Stützelements zu schützen.

2. Fußgelenkschiene nach Anspruch 1, wobei die Medialseitenstütze und die Lateralseitenstütze jeweils ein Polstermaterial (17, 18) aufweisen, das angrenzend an eine Innenfläche des jeweiligen steifen Stützelements angeordnet ist.

3. Fußgelenkschiene nach Anspruch 1, wobei die Umfangsrandabschnitte durch einen Klebstoff an den jeweiligen steifen Stützelementen haften.

4. Fußgelenkschiene nach Anspruch 3, wo b e i die Umfangsrandabschnitte an einer äußeren Stirnfläche der Medialseitenstütze bzw. der Lateralseitenstütze haften.

5. Fußgelenkschiene nach Anspruch 1 , wobei das Polstermaterial die Seitenränder der jeweiligen steifen Stützelemente überlappt und sich nach außen über diese hinaus erstreckt un d die Umfangsrandabschnitte die Seitenränder sowohl der steifen Stützelemente als auch des Polstermaterials überlappen und sich nach außen über diese hinaus erstrecken.

6. Fußgelenkschiene nach Anspruch 1, 2, 3, 4 oder 5, wobei das Medialstützelement und das Lateralstützelement jeweils aus einem vorgeformten Kunststoffmaterial gebildet sind.

7. Fußgelenkschiene nach Anspruch 5, wobei:
(a) das Polstermaterial die Seitenränder der steifen Stützelemente überlappt und sich nach außen über diese hinaus erstreckt,
(b) die Umfangsrandabschnitte die Seitenränder sowohl des steifen Stützelements als auch de s Polstermaterials überlappen und sich nach außen über diese hinaus erstrecken und
(c) die Umfangsrandabschnitte sowohl an de r steifen Seitenstütze als auch an den überlappten Abschnitten des Polstermaterials haften.

8. Fußgelenkschiene nach Anspruch 6, wobei das Polstermaterial ein dreidimensionales Gewebe aufweist.

9. Fußgelenkschiene na c h Anspruch 6, wobei das Polstermaterial zwei übereinander gelegte Schichten eines dreidimensionalen Gewebes aufweist.

10. Fußgelenkschiene nach Anspruch 6, wobei das Polstermaterial mindestens eine Schicht eines dreidimensionalen Gewebes des Typs aufweist, der **dadurch gekennzeichnet ist, dass** er sehr luftdurchlässig und frei von Latex ist.

11. Fußgelenkschiene nach Anspruch 1, welche mindestens einen Riemen zum Halten der Medialseitenstütze und der Lateralseitenstütze in einem eng übereinstimmenden Zustand anliegend an das untere Bein und das Fußgelenk aufweist.

12. Fußgelenkschiene nach Anspruch 1 0 , wobei das Polstermaterial zwischen 6 und 12 mm dick ist.

13. Fußgelenkschiene na c h Anspruch 10, wobei das Polstermaterial 9 mm dick ist.

14. Fußgelenkschiene nach Anspruch 2, wobei das Polstermaterial aufweist:
(a) eine Einzelschicht, die über der Innenfläche der steifen Stützelemente liegt, und
(b) eine zweite Schicht, die über einem Bereich des steifen Stützelements liegt, der neben dem Fußgelenk liegen und dieses stützen soll.

15. Fußgelenkschiene nach Anspruch 14, wobei das Polstermaterial ein dreidimensionales Gewebe aufweist.

16. Fußgelenkschiene nach Anspruch 1, wobei die Verbindungsmittel aufweisen:
(a) einen medialen Riemen, der von dem Medialstützelement getragen wird,
(b) einen lateralen Riemen, der von dem Lateralstützelement getragen wird, und
(c) komplementäre Befestigungselemente, die von dem medialen bzw. dem lateralen Riemen getragen werden, um zu ermöglichen, dass der mediale Riemen und der laterale Riemen lösbar miteinander verbunden werden.

17. Fußgelenkschiene nach Anspruch 16, wobei die komplementären Befestigungselemente Klettverschlüsse aufweisen.

18. Fußgelenkschiene nach Anspruch 1, wobei die Verbindungsmittel aufweisen:
(a) einen Riemen, der von einem von dem Medialstützelement und dem Lateralstützelement getragen wird, welcher ein von einem seiner Enden getragenes Befestigungselement aufweist, und
(b) ein komplementäres Befestigungselement, das von einer Außenfläche des anderen von dem Medialstützelement und dem Lateralstützelement getragen wird und vorgesehen ist, mit dem Befestigungselement zusammenzuwirken, um die Medialseitenstütze und die Lateralseitenstütze miteinander zu verbinden.

19. Fußgelenkschiene nach Anspruch 1, wobei der Umfangsrandabschnitt aus de r aus Vinyl, Gummi oder Kunstgummi bestehenden Gruppe ausgewählt ist.

20. Fußgelenkschiene nach Anspruch 1, wobei sich der Umfangsrandabschnitt zu seinem Außenrand verjüngt, um die Flexibilität zu erhöhen.

## Revendications

1. Attelle de cheville apte à s'étendre sur les faces latérale et interne d'une cheville blessée et de la partie inférieure d'une jambe afin de maintenir la cheville pendant le processus de guérison, et comprenant :
un support (11) côté interne destiné à s'étendre sur la face interne de la partie inférieure de la jambe,
un support (12) côté latéral destiné à s'étendre sur la face latérale de la cheville et de la partie inférieure de la jambe, et
des moyens d'accouplement (19, 20) pour réunir le support côté interne et le support côté latéral au niveau de leurs extrémités inférieures respectives, et s'étendant sous le pied du porteur de l'attelle ;
***caractérisée en ce que***
(a) le côté interne comprend un élément de soutien rigide (13) ayant une extrémité inférieure de largeur et d'épaisseur suffisamment faibles pour tenir à l'intérieur d'une chaussure portée par le porteur de l'attelle, ledit support côté interne comprenant une portion de bordure périmétrique souple et douce (15) adhérant mécaniquement et s'étendant vers l'extérieur dans une relation de chevauchement non emboîtante avec l'élément de soutien rigide afin de protéger la face interne de la partie inférieure de la jambe et de la cheville d'une irritation résultant du contact avec les bords de l'élément de soutien rigide, et
(b) le support côté latéral comprend un élément de soutien allongé rigide (14) ayant une extrémité inférieure de largeur et d'épaisseur suffisamment faibles pour tenir à l'intérieur de la chaussure, ledit support côté latéral comprenant une portion de bordure périmétrique souple et douce (16) adhérant mécaniquement et s'étendant vers l'extérieur dans une relation de chevauchement non emboîtante avec l'élément de soutien rigide afin de protéger la face latérale de la partie inférieure de la jambe et de la cheville d'une irritation résultant du contact avec les bords de l'élément de soutien rigide.

2. Attelle de cheville selon la revendication 1, dans laquelle le support côté interne et le support côté latéral comprennent chacun un matériau de rembourrage (17, 18) placé de manière adjacente à une face intérieure des éléments de soutien rigides respectifs.

3. Attelle de cheville selon la revendication 1, dans laquelle les portions de bordure périmétrique adhèrent aux éléments de soutien rigides respectifs grâce à un adhésif.

4. Attelle de cheville selon la revendication 3, dans laquelle les portions de bordure périmétrique adhèrent à une surface orientée vers l'extérieur des supports côté interne et côté latéral respectifs.

5. Attelle de cheville selon la revendication 1, dans laquelle le matériau de rembourrage chevauche et s'étend vers l'extérieur au-delà des bords latéraux des éléments de soutien rigides respectifs, et les portions de bordure périmétrique chevauchent et s'étendent vers l'extérieur au-delà des bords latéraux à la fois des éléments de soutien rigides et du matériau de rembourrage.

6. Attelle de cheville selon la revendication 1, 2, 3, 4 ou 5, dans laquelle l'élément de soutien interne et l'élément de soutien latéral sont l'un et l'autre formés d'un matériau plastique préformé.

7. Attelle de cheville selon la revendication 5, dans laquelle :
(a) ledit matériau de rembourrage chevauche et s'étend vers l'extérieur au-delà des bords latéraux des éléments de soutien rigides ;
(b) lesdites portions de bordure périmétrique chevauchent et s'étendent vers l'extérieur au-delà des bords latéraux à la fois de l'élément de soutien rigide et du matériau de rembourrage ; et
(c) lesdites portions de bordure périmétrique adhèrent à la fois au support latéral rigide et aux portions chevauchées du matériau de rembourrage.

8. Attelle de cheville selon la revendication 6, dans laquelle ledit matériau de rembourrage comprend un tissu tridimensionnel.

9. Attelle de cheville selon la revendication 6, dans laquelle ledit matériau de rembourrage comprend deux couches superposées d'un tissu tridimensionnel.

10. Attelle de cheville selon la revendication 6, dans laquelle ledit matériau de rembourrage comprend au moins une couche d'un tissu tridimensionnel du type **caractérisé par le fait qu'**il est fortement perméable à l'air et exempt de latex.

11. Attelle de cheville selon la revendication 1, et comprenant au moins une sangle pour maintenir contre la partie inférieure de la jambe et la cheville, le support côté interne et le support côté latéral dans une situation d'adaptation étroite.

12. Attelle de cheville selon la revendication 10, dans laquelle ledit matériau de rembourrage est d'une épaisseur comprise entre 6 et 12 mm.

13. Attelle de cheville selon la revendication 10, dans laquelle ledit matériau de rembourrage est d'une épaisseur de 9 mm.

14. Attelle de cheville selon la revendication 2, dans laquelle le matériau de rembourrage comprend :
(a) une simple couche recouvrant la face interne des éléments de soutien rigides ; et
(b) une deuxième couche recouvrant une zone de l'élément de soutien rigide adaptée pour se trouver à côté de la cheville et la maintenir.

15. Attelle de cheville selon la revendication 14, dans laquelle le matériau de rembourrage comprend un tissu tridimensionnel.

16. Attelle de cheville selon la revendication 1, dans laquelle lesdits moyens d'accouplement comprennent :
(a) une sangle interne portée par l'élément de soutien interne ;
(b) une sangle latérale portée par l'élément de soutien latéral ; et
(c) des éléments d'attache complémentaires portés respectivement par la sangle interne et la sangle latérale, afin de permettre à la sangle interne et à la sangle latérale d'être accouplée/réunies de manière libérable.

17. Attelle de cheville selon la revendication 16, dans laquelle lesdits éléments d'attache complémentaires comprennent des unités de fermeture auto-agrippante.

18. Attelle de cheville selon la revendication 1, dans laquelle lesdits moyens d'accouplement comprennent :
(a) une sangle portée par l'un d'entre l'élément de soutien interne et l'élément de soutien latéral ayant un élément d'attache porté sur une extrémité de celui-ci,
(b) un élément d'attache complémentaire porté sur une surface externe de l'autre d'entre l'élément de soutien interne et l'élément de soutien latéral, destiné à coopérer avec l'élément d'attache pour accoupler le support côté interne et le support côté latéral.

19. Attelle selon la revendication 1, dans laquelle ladite portion de bordure périmétrique est choisie dans le groupe constitué du vinyle, du caoutchouc et du caoutchouc synthétique.

20. Attelle selon la revendication 1, dans laquelle ladite portion de bordure périmétrique s'amincit en direction de son rebord externe afin d'accroître sa souplesse.
